# EUROPÄISCHE PATENTANMELDUNG

(11) **EP 4 725 397 A1**
(43) Veröffentlichungstag der Anmeldung: **15.04.2026**
(21) Anmeldenummer: 25206840.8
(22) Anmeldetag: 06.10.2025
(51) Int. Cl.: A61B 5/00, G01N 21/65, G01J 3/44, G01N 33/02

(54) **MOBILES RAMAN-SPEKTROMETER-SYSTEM UND VERFAHREN FÜR DIE MEDIZINISCHE DIAGNOSTIK VON MENSCHLICHEN, TIERISCHEN ODER PFLANZLICHEN PROBEN**

(30) Priorität: 09.10.2024 DE 102024129205
(71) Anmelder: Netzsch-Gerätebau GmbH, 95100 Selb (DE)
(72) Erfinder: Küspert, Sabrina, 95100 Selb (DE); Rummel, Florian, 95100 Selb (DE); Hollering, Markus, 95632 Wunsiedel (DE); Taubmann, Rebekka, 95100 Selb (DE)

(57) **Zusammenfassung**

Die vorliegende Erfindung betrifft ein mobiles Raman-Spektrometer-System (1) für die medizinische Diagnostik von menschlichen, tierischen oder pflanzlichen Proben. Solch ein System (1) umfasst dabei eine Messeinrichtung (2), eine damit verbundene Auswerteeinheit (3) mit Auswerteprogramm (4) und eine damit verbundene Ausgabeeinheit (8). Dabei ist die Auswerteeinheit (3) mit Auswerteprogramm (4) ausgelegt, die gemessenen Raman-Spektrometer-Parameter einer menschlichen, tierischen oder pflanzlichen Probe hinsichtlich wenigstens einer medizinischen Diagnose zu klassifizieren, sodass einem Anwender des mobilen Raman-Spektrometer-Systems (1) eine differenzierte Ansicht der Probe mittels der Ausgabeeinheit (8) bereitstellbar ist. Zudem wird ein entsprechendes Verfahren (M) vorgestellt.

## Beschreibung

### TECHNISCHES GEBIET DER ERFINDUNG

Die vorliegende Erfindung betrifft ein mobiles Raman-Spektrometer-System sowie ein Verfahren für die medizinische Diagnostik von menschlichen, tierischen oder pflanzlichen Proben.

### HINTERGRUND DER ERFINDUNG

Um weitergehende Aussagen über menschliche, tierische oder pflanzliche Proben treffen zu können, werden diese häufig nach der Entnahme vom Organismus aufwendig im Labor analysiert. Alternativ ist auch bekannt, eine im Zusammenhang mit dem zu untersuchendem Gewebe oder Organismus stehende Flüssigkeit zu untersuchen.

So werden beispielsweise Proben von einem Menschen während einer Biopsie oder einer Operation durch medizinisches Fachpersonal entnommen und anschließend zur Pathologie gebracht. In der Pathologie erfolgt anschließend häufig eine histologische Untersuchung, typischerweise mit Färbung von Gewebe und nachfolgender pathologischer Diagnostik. Auch können Gewebeproben mittels Gewebeschnitte aufbereitet werden, um diese einzelnen Schnitte jeweils anschließend weiter zu analysieren.

Die skizzierten Vorgehensweisen mit den jeweiligen einzusetzenden Techniken gehen mit einem gewissen Zeitaufwand einher. Zudem wird für den Transport der Proben eine bestimmte Logistikinfrastruktur benötigt, um die häufig sensiblen Proben während dieser Zeit nicht zu beschädigen. In diesem Zusammenhang sind trotz aller Vorsichtsmaßnahmen Veränderungen der Proben wahrscheinlich und können nicht gänzlich ausgeschlossen werden. Generell ändert sich biologisches Probenmaterial zeitlich schnell, sodass nachfolgende Untersuchungen beziehungsweise die Analytik erschwert wird.

Für die oben erwähnten Fälle der Biopsie und der Entnahme während einer Operation ist die nötige medizinische Infrastruktur häufig nur bei Maximalversorgern gegeben. Eine Versorgung und Diagnostik in entlegenen Gegenden, wie beispielsweise im Hochgebirge oder auf hoher See, kann sich somit als schwierig erweisen, da die nötige medizinische Infrastruktur entweder nur schwer bereitstellbar oder schlicht nicht gegeben ist.

Es können sich zudem Risiken ergeben, wenn ein Chirurg nur per Auge im Zusammenhang mit dem bis dahin vorliegenden Befund entscheidet, wo ein Schnitt zur Entfernung von Tumorgewebe beziehungsweise des gänzlichen Tumors zu setzen beziehungsweise durchzuführen ist. Falls durch den Schnitt das Tumorgewebe getroffen wird, besteht eine höhere Wahrscheinlichkeit, dass der Tumor im Körper streut, da sich die Krebszellen nun leichter weiter verteilen und damit eine weitere Schädigung im Körper verursachen können. Ähnlich ist die Situation bei einem Verdacht auf Hautkrebs. Auch hier werden aktuell Proben entnommen und anschließend an eine Laboranalytik weitergeleitet.

Bekannte bildgebende Verfahren bedingen häufig größere Apparaturen, die nur in einer speziellen Umgebung, beispielsweise in einem für diese Zwecke ausgestalteten Anwendungsraum, zum Einsatz kommen können. Zudem ist es meist nicht ohne weiteres möglich, gleichzeitig den eigentlichen chirurgischen Eingriff vorzunehmen, da entweder aus Sicherheitsgründen oder schlicht aus Platzmangel solche parallelen Vorgänge nicht durchführbar sind.

### ZUSAMMENFASSUNG DER ERFINDUNG

Vor diesem Hintergrund liegt der vorliegenden Erfindung die Aufgabe zugrunde, ein System und ein Verfahren bereitzustellen, welche zumindest teilweise die zuvor genannten Probleme und Nachteile überkommen.

Diese Aufgabe wird gelöst durch ein mobiles Raman-Spektrometer-System mit den Merkmalen des Patentanspruchs 1 sowie durch ein Verfahren mit den Merkmalen des Patentanspruchs 13.

Dementsprechend ist ein mobiles Raman-Spektrometer-System für die medizinische Diagnostik von menschlichen, tierischen oder pflanzlichen Proben vorgesehen. Solch ein Raman-Spektrometer-System umfasst dabei eine Messeinrichtung, eine damit verbundene Auswerteeinheit mit Auswerteprogramm und eine damit verbundene Ausgabeeinheit. Die Auswerteeinheit mit Auswerteprogramm ist dabei ausgelegt, die gemessenen Raman-Spektrometer-Parameter einer menschlichen, tierischen oder pflanzlichen Probe hinsichtlich wenigstens einer medizinischen Diagnose zu klassifizieren, sodass einem Anwender des mobilen Raman-Spektrometer-Systems eine differenzierte Ansicht der Probe mittels der Ausgabeeinheit bereitstellbar ist.

Des Weiteren ist ein Verfahren für die medizinische Diagnostik von menschlichen, tierischen oder pflanzlichen Proben vorgesehen. Solch ein Verfahren umfasst dabei die folgenden Schritte: Bereitstellen und Aktivieren eines erfindungsgemäßen mobilen Raman-Spektrometer-Systems; Messen einer menschlichen, tierischen oder pflanzlichen Probe mittels des erfindungsgemäßen mobilen Raman-Spektrometer-Systems; Klassifizieren von Ergebnissen der Raman-Spektrometer-Messungen hinsichtlich wenigstens einer medizinischen Diagnose mittels des erfindungsgemäßen mobilen Raman-Spektrometer-Systems; Ausgeben der klassifizierten Ergebnisse mittels des erfindungsgemäßen mobilen Raman-Spektrometer-Systems, sodass einem Anwender des erfindungsgemäßen mobilen Raman-Spektrometer-Systems eine differenzierte Ansicht der Probe mittels des erfindungsgemäßen mobilen Raman-Spektrometer-Systems bereitstellbar ist.

Eine der Erfindung zugrunde liegende Idee besteht in der Verwendung der Raman-Spektroskopie für die medizinische Diagnostik von menschlichen, tierischen oder pflanzlichen Proben. Dabei ist zudem vorgesehen, dass solch ein Einsatz parallel zu einer eigentlichen Behandlung der Probe gewährleistbar ist, falls dies wünschenswert ist und nötig erscheint. Die Raman-Spektroskopie nutzt die Wechselwirkung von Licht und Materie. Damit sind Einblicke in den molekularen Aufbau und die Eigenschaften eines Materials, beispielsweise in Form von menschlichen Gewebe, möglich.

Es ist somit vorgesehen, dieses Prinzip der Raman-Spektroskopie zu nutzen, um beispielsweise während eines chirurgischen Eingriffs unter zu Hilfenahme des erfindungsgemäßen Systems zwischen gesunden und kranken Gewebe unterscheiden zu können. Anstatt einer aufwendigen Entnahme einer Probe und eines anschließenden zeitlich aufwendigen Transports zu einer Laboranalytik, welche womöglich sehr weit entfernt von der eigentlichen Probeentnahmestelle liegt, ist somit eine Diagnostik direkt vor Ort unter Verwendung des erfindungsgemäßen mobilen Raman-Spektrometer-Systems möglich. Es kann somit direkt vor Ort vom Fachpersonal ein Einsatz mit Hilfe des vorgestellten Systems durchgeführt werden, sodass während der Sichtung der Probe selbst bereits weitergehende Aussagen über Zustände der Probe in Echtzeit zur Verfügung gestellt werden können.

Dabei ist ein Einsatz des mobilen Raman-Spektrometer-Systems sowohl innerhalb eines Operationssaals als auch außerhalb dieses Saals möglich. Im Gegensatz zu Bildgebungsverfahren, die einen sehr großen Platzbedarf für die jeweiligen Komponenten der Gerätschaft benötigen, bietet das vorgestellte System die Möglichkeit einer mobilen Anwendung. Mit anderen Worten ist das vorgestellte System so kompakt ausgebildet, sodass auf vorteilhafte Weise ein mobiler Einsatz auch außerhalb von einer speziellen Umgebung, beispielsweise in Form eines Maximalversorgers oder einer krankenhausähnlichen Umgebung, möglich ist.

Aufgrund der mobilen Anwendungsmöglichkeit des vorgesehenen Systems entfällt der ansonsten nötige Zeitaufwand für aufwendige Transporte zur Laboranalytik, sodass die Nutzen der Diagnostik unmittelbar an Ort und Stelle einem Fachpersonal zur Verfügung stehen. Zudem benötigt das vorgestellte System keine gesonderte Markierung der Probe. Bei der Raman-Spektroskopie ist eine Markierung von Proben, beispielsweise in Form von Zellen oder Zellgewebe, nicht nötig, wie sie etwa bei anderen spektroskopischen Methoden notwendig sein kann.

Das vorgestellte System kann unmittelbar am Organismus, welcher menschlicher, tierische oder pflanzlicher Natur sein kann, eingesetzt werden, sodass ein Schnitt, etwa in Form eines Schnellschnitts, welcher für die anschließende Histologie während eines operativen Eingriffs oder generell während einer Probeentnahme andernfalls nötig wäre, entfallen kann. Somit bietet sich aufgrund des vorgestellten Systems beziehungsweise des zugehörigen Verfahrens der Vorteil, dass es einem Chirurgen oder allgemein einer Person, welche sich mit einer zu untersuchenden Probe beschäftigt, die Möglichkeit, nur in gesundes Gewebe zu schneiden oder allgemein in Stellen von Proben zu schneiden, welche als unauffällig und aufgrund der durchgeführten Klassifizierung als gesund und nicht negativ behaftet gelten.

Durch die vorliegende Erfindung ist es einem Chirurgen vorteilhaft möglich, nur in gesundes Gewebe zu schneiden und auf diese Weise etwa einen Tumor vollständig zu entfernen, ohne dabei gesundes Gewebe zu beschädigen. Insofern kann die vorliegende Erfindung dazu beitragen, das Risiko zu vermindern, dass der Tumor aufgrund von Einschnitten in tumoröses Gewebe weiter streut. Eine Verschlechterung einer Erkrankung kann also weiter minimiert werden, insbesondere das Risiko von weiteren Streuungen von Tumorzellen aufgrund von ungewünschten Einschnitten in tumoröses Gewebe. Dies kann parallel zur Messung geschehen, da die klassifizierten Messergebnisse dem Chirurgen in Echtzeit während des Eingriffes zur Verfügung stehen.

Auch kann die vorliegende Erfindung bei einer Untersuchung mit Vorteil eingesetzt werden, welche lediglich basierend auf einer Verdachtsdiagnose nötig erscheint. Bislang musste hierzu eine Gewebeprobe entnommen werden und eingeschickt werden. Mit dem erfindungsgemäßen System ist es dem Facharzt möglich, direkt beim ersten Patientenbesuch eine mögliche Erkrankung auszuschließen oder im Fall der Erkrankung sofort notwendige Behandlungsschritte einzuleiten. Dies kann die Behandlung entscheidend beschleunigen und so einen entscheidenden Zeitvorteil darstellen, um bei schnell voranschreitenden Krankheiten eine Überlebenschance zu ermöglichen.

Die Probe kann in Form von Gewebe vorliegen, wobei sie menschlichen, tierischen oder pflanzlichen Ursprungs sein kann. Auch kann sie in jeglichem Zusammenhang mit diesem Gewebe oder Organismus stehen.

Das erfindungsgemäße mobile Raman-Spektrometer-System kann in diesem Zusammenhang auch außerhalb eines Operationssaals mit Vorteil verwendet werden, um zum Beispiel Messungen an der Haut eines Organismus oder an Hautgewebe eines Organismus oder an anderem Gewebe eines Organismus vorzunehmen.

Zum Beispiel kann das mobile Raman-Spektrometer-System mit Vorteil ausgelegt sein, aufgrund seiner handlichen Ausmaße von Hautärzten bei Verdacht auf Hautkrebs eingesetzt zu werden oder es kann mit Vorteil allgemein bei Patienten eingesetzt werden, welche eine mögliche Nachverfolgung / Kontrolle ihres Gesundheitszustandes bedürfen. Gleichzeitig kann das vorgestellte mobile Raman-Spektrometer-System auch als Teil einer Ausstattung von Operationssälen genutzt werden.

Das erfindungsgemäße mobile Raman-Spektrometer-System kann ebenfalls mit Vorteil in der Toxikologie und in der Pharmakologie eingesetzt und verwendet werden. Zudem kann das erfindungsgemäße mobile Raman-Spektrometer-System bei Untersuchungen von Materialzusammensetzungen eingesetzt werden, beispielsweise, um Unterschiede festzustellen. Dies kann etwa mit Vorteil bei der Begutachtung oder Untersuchung von Implantaten oder dergleichen vorgesehen sein.

Gemäß eines Ausführungsbeispiel des mobilen Raman-Spektrometer-Systems ist vorgesehen, dass die Auswerteeinheit mit Auswerteprogramm für die Zwecke der Klassifizierung mit wenigstens einer Datenbank für die medizinische Diagnostik von menschlichen, tierischen oder pflanzlichen Proben basierend auf den Raman-Spektrometer-Parametern verbindbar ist oder wenigstens eine solche Datenbank umfasst.

Auf diese Weise ist es möglich, ein besonders flexibles System hervorzubringen, welches zudem die Möglichkeit bietet, über die in der Auswerteeinheit hinterlegten Daten hinaus eine noch umfangreichere Klassifizierung zu ermöglichen. Dabei kann es möglich sein, Zugriffe auf externe Datenbanken zu ermöglichen oder Zugriffe auf Datenbanken zu ermöglichen, welche direkt ein Teil des erfindungsgemäßen Systems sind.

Mit anderen Worten kann das vorgestellte System wenigstens eine Datenbank beinhalten oder mit einer solchen verknüpft sein, die somit einem medizinischen Fachpersonal direkt während oder nach der Messung eine Auswertung und eine damit verbundene Diagnose ermöglicht. Dabei kann eine Anwendung auch in Echtzeit vorgesehen sein.

Gemäß einer Weiterbildung des mobilen Raman-Spektrometer-Systems ist vorgesehen, dass das Auswerteprogramm mittels wenigstens einer benutzerdefinierten Eingabe anpassbar ist, sodass eine individualisierte Nutzung der jeweiligen Datenbank möglich ist.

Somit kann eine noch gezieltere Anwendung des vorgestellten Systems mit Vorteil gewährleistet werden. Die Eingabe kann mit Vorteil genutzt werden, sodass die jeweiligen verknüpften Datenbanken oder auch nur eine Datenbank bestmöglich für die gewünschte Diagnose eingesetzt werden kann.

Gemäß einer Weiterbildung des mobilen Raman-Spektrometer-Systems ist vorgesehen, dass die wenigstens eine benutzerdefinierte Eingabe ausgewählt ist aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden oder dergleichen, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.

Je nach Auswahl kann die zuvor erwähnte speziellere Anwendung des vorgestellten Systems noch besser bereitgestellt werden. Auch kann somit eine gezieltere Unterstützung einer medizinischen Forschung oder einer Forschung an pflanzlichen Organismen ermöglicht werden. Beispielsweise kann das System mit Vorteil so ausgelegt werden, sodass es grundsätzlich gendersensibel auswertet und auf diese Weise insbesondere eine medizinische Forschung oder eine Forschung an pflanzlichen Organismen unterstützt, die auf geschlechterspezifische Unterschiede Rücksicht zu nehmen hat.

Die wenigstens eine Information über korrelierende Einflüsse von Medikamenten und pathologischen Veränderungen bei wenigstens einem Organismus kann beispielsweise im Sinne der Erkenntnis vorliegen, wie sie in Indien zwischen dem Einsatz von Diclofenac bei Nutztieren und dem Geiersterben auftraten. Aufgrund der Abgabe von Diclofenac an Nutztieren gelang über die Aufnahme des Aas diese Medikament in den jeweiligen Organismus der dortigen Geierpopulation, was ein Geiersterben zur Folge hatte, da dieses Medikament für Geier unverträglich ist.

Es ist somit möglich, dass eine jeweilige Datenbank mittels der Eingabe mit weiteren Daten, wie zum Beispiel einer Ernährung oder Medikation eines Patienten, versehen beziehungsweise ergänzt wird, sodass entsprechend korrelierende Verfahren oder Korrekturen mit Vorteil vornehmbar sind, um beispielsweise Einflüsse von Faktoren zu korrigieren, die nicht notwendigerweise oder alleinig mit einer zu diagnostizierenden Krankheit einhergehen müssen.

In diesem Zusammenhang ist es vorstellbar, dass bereits erste Daten über die zuvor genannten Umstände in der jeweiligen Datenbank vorhanden sind und die weiteren Eingaben beispielsweise auch als benutzerdefinierte Anpassung der Datenbank im Sinne einer Aktualisierung mit neusten Daten vorteilhaft in Echtzeit vornehmbar sind.

Gemäß eines Ausführungsbeispiels des mobilen Raman-Spektrometer-Systems ist vorgesehen, dass die Messeinrichtung ausgelegt ist, Raman-Spektrometer-Parameter der menschlichen, tierischen oder pflanzlichen Proben separat oder am Organismus zu messen.

Ein flexibler Einsatz des mobilen Raman-Spektrometer-Systems kann somit auf vorteilhafte Weise gewährleistet werden. Insbesondere ein Einsatz direkt am Organismus kann ein Teil der zuvor genannten Vorteile noch besser unterstützen.

So kann vorgesehen sein, die entsprechende Komponente des mobilen Raman-Spektrometer-Systems direkt auf die Probe, etwa in Form von zu untersuchendem Gewebe, anzulegen beziehungsweise diese Komponente, etwa die Messeinrichtung, direkt davorzuhalten.

Insbesondere kann somit mit Vorteil die entsprechende Komponente des mobilen Raman-Spektrometer-Systems, etwa die Messeinrichtung, bei einem Facharzt direkt auf die zu bemessende Probe, etwa in Form von Zellgewebe oder allgemein von Gewebe anzulegen beziehungsweise die entsprechende Komponente des mobilen Raman-Spektrometer-Systems direkt vor das zu bemessende beziehungsweise zu untersuchende Gewebe zu halten, ohne dass es eines operativen Eingriffs bedarf.

Dies kann somit in Echtzeit beziehungsweise instantan am Gewebe beziehungsweise am zu untersuchenden Organismus geschehen. Die Untersuchung mit dem erfindungsgemäßen System erfolgt somit in unmittelbarer räumlicher und zeitlicher Nähe. Mit anderen Worten ist das vorgestellte erfindungsgemäße System ausgebildet, für einen direkten Einsatz am zu untersuchenden Organismus beziehungsweise an Teilbereichen des zu untersuchenden Organismus eingesetzt zu werden.

Insbesondere können somit Bestandteile, etwa die Messeinrichtung, nicht nur in dem Gewebe, sondern auch am Gewebe und somit vor der Entnahme einer Gewebe- oder Materialprobe verwendet werden. Somit bleibt das zu untersuchende Gewebe intakt und wird gegebenenfalls nur dann entfernt, wenn dieser Vorgang aufgrund einer mittels des Systems erstellten oder wenigstens eine mit dem System unterstützend erstellte Diagnose dies notwendig erscheinen lässt.

Gemäß eines Ausführungsbeispiels des mobilen Raman-Spektrometer-Systems ist vorgesehen, dass das mobile Raman-Spektrometer-System ein Energiesystem umfasst, welches ausgelegt ist, das Raman-Spektrometer-System autark oder mittels eines externen Energieversorgungsnetzes mit elektrischer Energie zu versorgen.

Eine autarke Energieversorgung mit einem aufladbarem, eigenständigem Energiesystem unterstützt die portable Funktionsweise des vorgestellten Systems auf vorteilhafte Weise. Der mobile Einsatz des vorgestellten erfindungsgemäßen Raman-Spektrometer-Systems ist somit noch besser gewähr leistbar. Beispielsweise kann ein zu untersuchender Organismus auch außerhalb eines Operationssaals somit mit Vorteil schnell und effizient untersucht werden, um eine Erstellung einer folgerichtigen Diagnose in Echtzeit vor Ort zu ermöglichen.

Da auch ein Netzbetrieb möglich ist, bietet das vorgestellte System somit auch die Möglichkeit, dass das System Teil einer größeren Infrastrukturumgebung etwa eines Operationssaal sein kann, wobei etwa eine zentrale Energieversorgungsvorrichtung für einen reibungslosen Ablauf vorgehalten und betrieben werden kann.

Mit anderen Worten kann das vorgestellte System sowohl Netz- als auch Batteriebetrieben verwendet werden, um sowohl in einem stationären Betriebsmodus als auch in einem mobilen Betriebsmodus betrieben zu werden. Insbesondere im mobilen Betriebsmodus ist das System somit geeignet, auch in entlegeneren Gegenden ohne nennenswerte Infrastruktur mit Vorteil betrieben beziehungsweise verwendet werden zu können.

Gemäß einer Weiterbildung des mobilen Raman-Spektrometer-Systems ist vorgesehen, dass das Raman-Spektrometer-System im Wesentlichen Außenmaterialien, insbesondere Edelstahl, umfasst, welche für ein Sterilisationsverfahren geeignet sind, sodass das mobile Raman-Spektrometer-System nach Durchlauf eines Sterilisierverfahrens in einem Operationssaal einsetzbar ist.

Insofern kann das vorgestellte System mit Vorteil direkt dort angewandt werden, wo eine hygienisch einwandfreie Umgebung zwingend einzuhalten ist. Insofern kann das System hygienisch designt und ausgelegt sein. Insbesondere eine zumindest teilweise Einhausung mit geeigneten Materialien, etwa mit Edelstahl oder dergleichen, kann diesen Vorteil besonders effektiv gewährleisten.

Gemäß eines Ausführungsbeispiels des mobilen Raman-Spektrometer-Systems ist vorgesehen, dass das Raman-Spektrometer-System ausgelegt ist, eine Vielzahl von Proben simultan zu messen und zu klassifizieren.

Auf diese Weise ist ein besonders effizienter Einsatz des vorgestellten Systems möglich. Beispielsweise können somit mit Vorteil benachbarte Gewebebereiche an einem zu untersuchenden Organismus bemessen beziehungsweise jeweilige kritische Bereiche gemessen werden.

Gemäß eines Ausführungsbeispiels des mobilen Raman-Spektrometer-Systems ist vorgesehen, dass das Raman-Spektrometer-System ausgelegt ist, mittels wenigstens einem mit dem Raman-Spektrometer-System koppelbaren externen Gerät gesteuert zu werden.

Insbesondere für den Einsatz in entlegeneren Gebieten kann somit ein besonders flexibel einsetzbares System bereitgestellt werden. Auch Fachpersonal, was nicht alle Bedienweisen eines komplex ausgestalteten Operationssaals beherrscht, kann somit auf vorteilhafte Art und Weise unmittelbar eine Anwendung des Systems mit einfachen Mitteln durchführen. Die jeweiligen externen Geräte können hierzu etwa eine gesonderte Applikation aufweisen, welche beispielsweise von dem System bereitstellbar ist.

Auch ist vorstellbar, dass die Auswerteeinheit mit Auswerteprogramm ausgelegt ist, auf dem externen Gerät gespiegelt zu werden, sodass eine unmittelbare Steuerung und Bedienweise des erfindungsgemäßen mobilen Raman-Spektrometer-Systems mittels des Auswerteeinheit mit Auswerteprogramm via dem jeweiligen externen Gerät möglich ist.

Gemäß eines Ausführungsbeispiels des mobilen Raman-Spektrometer-Systems ist vorgesehen, dass das externe Gerät auswählbar ist aus: Smartphone, Tablet, Smartwatch, Laptop, Cloud-Anwendung. Die zuvor genannten Vorteile sind somit noch gezielter und anwenderfreundlicher zu erreichen..

Gemäß eines Ausführungsbeispiels des mobilen Raman-Spektrometer-Systems ist vorgesehen, dass die Messeinrichtung weitere Verbindungsmittel zur Kopplung der Messeinrichtung mit der Auswerteeinheit mit Auswerteprogramm umfasst, welche ausgelegt sind, die Messeinrichtung separat von den restlichen Komponenten des mobilen Raman-Spektrometer-Systems im Umkreis des mobilen Raman-Spektrometer-Systems in einem Abstandsintervall von 0,1 bis 10 m, vorzugsweise von 0,5 bis 5 m, vorzugsweise von 1 bis 3 m, einzusetzen, sodass die mittels der Messeinrichtung gemessenen Raman-Spektrometer-Parameter einer menschlichen, tierischen oder pflanzlichen Probe entsprechend in Bezug auf die restlichen Komponenten des mobilen Raman-Spektrometer-Systems örtlich flexibel erhebbar sind.

Ein flexibler Einsatz ist somit noch besser gewährleistbar. Insbesondere schwer zugängliche Bestandteile von zu untersuchenden Organismen können somit vorteilhaft untersucht werden, da die Messeinrichtung separat an der unzugänglichen Stelle optimal platziert werden kann, sodass Messungen nicht nur leichter, sondern auch besonders zielgenau durchführbar sind.

Gemäß eines Ausführungsbeispiels des mobilen Raman-Spektrometer-Systems ist vorgesehen, dass die Messeinrichtung im Wesentlichen stabförmig ausgebildet ist und einem Umfang aufweist, welcher mit einer Hand umgreifbar ist, wobei ein erster Endbereich ausgelegt ist, mit der zu bemessenden Probe in Kontakt gebracht zu werden, und ein zweiter Endbereich ausgelegt ist, wenigstens eine Strahlungsquelle austauschbar im Inneren der Messeinrichtung zu halten, sodass emittierte Strahlen der wenigstens eine Strahlungsquelle im Wesentlichen in Richtung erster Endbereich auslenkbar sind.

Das vorgestellte System ist somit besonders anwenderfreundlich und bedienerfreundlich anwendbar. Die stabförmige Ausbildung der Messeinrichtung kann etwa in der Größe eines Stiftes oder dergleichen vorgesehen sein, sodass ein Einsatz direkt am Gewebe oder auch an Körperflüssigkeiten mit darin zum Beispiel dispergierten oder gelösten Partikeln oder Tröpfchen oder Blasen oder Partikeln möglich ist. Auch ist ein Einsatz an sich im Körper des Organismus befindlichen aber potentiell körperfremden Materialien möglich. Ein Chirurg kann diese Messeinrichtung somit wie eine Art Sonde in der Größe eines Stiftes oder ähnlich einsetzen.

Gemäß eines Ausführungsbeispiels des erfindungsgemäßen Verfahrens ist vorgesehen, dass folgende weitere Verfahrensschritte vorgesehen sind: Verbinden der Auswerteeinheit mit Auswerteprogramm von dem mobilen Raman-Spektrometer-System mit wenigstens einer Datenbank für die medizinische Diagnostik von menschlichen, tierischen oder pflanzlichen Proben basierend auf den Raman-Spektrometer-Parametern; Anpassen des Auswerteprogramms mittels wenigstens einer benutzerdefinierten Eingabe, sodass eine individualisierte Nutzung der verbundenen externen Datenbank möglich ist.

Auf diese Weise ist es möglich, ein besonders flexibles Verfahren hervorzubringen, welches zudem die Möglichkeit bietet, über die in der Auswerteeinheit hinterlegten Daten hinaus eine noch umfangreicherer Klassifizierung zu ermöglichen. Dabei kann es möglich sein, Zugriffe auf externe Datenbanken zu ermöglichen oder Zugriffe auf Datenbanken zu ermöglichen, welche direkt ein Teil des erfindungsgemäßen Systems sind.

Mit anderen Worten kann das vorgestellte Verfahren vorsehen, dass das dabei einzusetzende erfindungsgemäße System wenigstens eine Datenbank beinhaltet oder mit einer solchen verknüpft ist, sodass somit einem medizinischen Fachpersonal direkt während oder nach der Messung eine Auswertung und eine damit verbundene Diagnose ermöglicht werden kann. Dabei kann eine Anwendung auch in Echtzeit vorgesehen sein.

Gemäß eines weiteren Ausführungsbeispiels des erfindungsgemäßen Verfahrens ist vorgesehen, dass die wenigstens eine benutzerdefinierte Eingabe ausgewählt ist aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden oder dergleichen, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.

Je nach Auswahl kann die zuvor erwähnte speziellere Anwendung des vorgestellten Verfahrens noch besser bereitgestellt werden. Auch kann somit eine gezieltere Unterstützung einer medizinischen Forschung oder einer Forschung an pflanzlichen Organismen ermöglicht werden. Beispielsweise kann das Verfahren mit Vorteil so ausgelegt werden, sodass es grundsätzlich gendersensibel auswertet und auf diese Weise insbesondere eine medizinische Forschung oder eine Forschung an pflanzlichen Organismen unterstützt, die auf geschlechterspezifische Unterschiede Rücksicht zu nehmen hat.

Es ist somit möglich, dass eine jeweilige Datenbank mit weiteren Daten, wie zum Beispiel einer Ernährung oder Medikation eines Patienten, versehen wird, sodass entsprechend korrelierende Verfahren oder Korrekturen mit Vorteil vornehmbar sind, um beispielsweise Einflüsse von Faktoren zu korrigieren, die nicht notwendigerweise oder alleinig mit einer zu diagnostizierenden Krankheit einhergehen müssen. Korrelierende Verfahren können zum Beispiel dann sinnvoll sein, wenn es darum geht, gewisse Einflüsse, zum Beispiel hormonelle Schwankungen im Zyklus der Frau, zu verdeutlichen, zu bedenken oder zu berücksichtigen.

In diesem Zusammenhang ist es vorstellbar, dass bereits erste Daten über die zuvor genannten Umstände in der jeweiligen Datenbank vorhanden sind und die weiteren Eingaben beispielsweise auch als benutzerdefinierte Anpassung der Datenbank im Sinne einer Aktualisierung mit neusten Daten vorteilhaft in Echtzeit vornehmbar sind.

Die wenigstens eine Information über korrelierende Einflüsse von Medikamenten und pathologischen Veränderungen bei wenigstens einem Organismus kann beispielsweise im Sinne der Erkenntnis vorliegen, wie sie in Indien zwischen dem Einsatz von Diclofenac bei Nutztieren und dem Geiersterben auftraten. Aufgrund der Abgabe von Diclofenac an Nutztieren gelang über die Aufnahme des Aas diese Medikament in den jeweiligen Organismus der dortigen Geierpopulation, was ein Geiersterben zur Folge hatte, da dieses Medikament für Geier unverträglich ist.

### KURZBESCHREIBUNG DER ZEICHNUNGEN

Im Folgenden wird die Erfindung unter Bezugnahme auf die Figuren der Zeichnungen erläutert. Von den Figuren zeigen:
- Fig. 1: eine schematische Darstellung eines mobilen Raman-Spektrometer-Systems nach einem Ausführungsbeispiel der vorliegenden Erfindung;
- Fig. 2: eine schematische Darstellung eines Anwenderszenarios eines mobilen Raman-Spektrometer-Systems nach einem Ausführungsbeispiel der vorliegenden Erfindung;
- Fig. 3: eine schematische Darstellung eines weiteren Anwenderszenarios eines mobilen Raman-Spektrometer-Systems nach einem Ausführungsbeispiel der vorliegenden Erfindung;
- Fig. 4: ein schematisches Flussdiagramm für ein Verfahren für die medizinische Diagnostik von menschlichen, tierischen oder pflanzlichen Proben nach einem Ausführungsbeispiel der vorliegenden Erfindung.

In den Figuren bezeichnen dieselben Bezugszeichen gleiche oder funktionsgleiche Komponenten, soweit nichts Gegenteiliges angegeben ist.

### AUSFÜHRLICHE BESCHREIBUNG VON AUSFÜHRUNGSBEISPIELEN DER ERFINDUNG

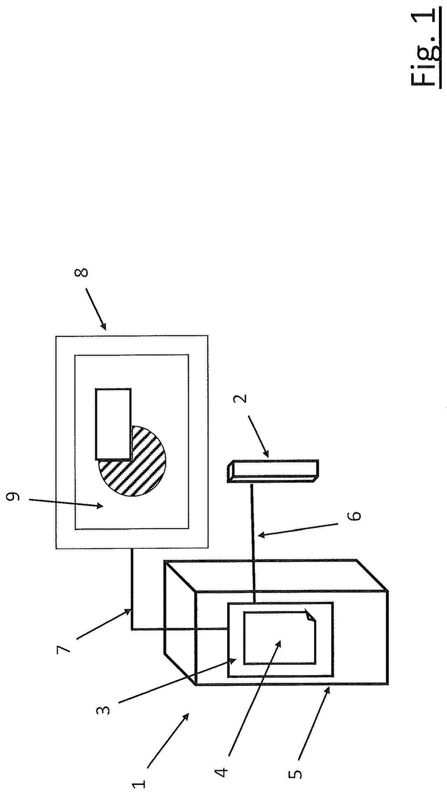

Fig. 1 zeigt eine schematische Darstellung eines mobilen Raman-Spektrometer-Systems 1 nach einem Ausführungsbeispiel der vorliegenden Erfindung. Dabei ist das mobile Raman-Spektrometer-System 1 für den mobilen Einsatz vorgesehen. Insbesondere ist es vorstellbar, dass jegliche Abmaße von einzelnen Komponenten des dargestellten mobilen Raman-Spektrometer-Systems 1 so bemessen sind, sodass ein einfacher Transport, beispielsweise von einer Person oder einer Drohne alleine, möglich ist.

Beispielsweise können die Abmaße so vorgesehen sein, sodass ein mobiles Raman-Spektrometer-System 1 in einer Bauform ähnlich eines Handheld-Gerätes resultiert, das mobil oder stationär in einem Operationssaal oder bei einem Facharzt in der Praxis verfügbar beziehungsweise einsetzbar ist.

Insofern ist in einer Vielzahl von möglichen Ausführungsvarianten des erfindungsgemäßen mobilen Raman-Spektrometer-Systems 1 vorstellbar, dass dieses System 1 zumindest anteilig portabel ausgebildet ist.

Das mobile Raman-Spektrometer-Systems 1 kann auch als ein medizinisches Gerät angesehen und so bezeichnet werden, welches mit Vorteil in einem medizinisches Kontext, beispielsweise bei der medizinische Diagnostik von menschlichen, tierischen oder pflanzlichen Proben einsetzbar und anwendbar ist. Optional kann das Raman-Spektrometer-System 1 mit einem chirurgischen Werkzeug bestückt werden, um die räumliche Nähe zwischen zu untersuchendem/ zu schneidenden/ zu entfernenden Gewebe und des durch die Messung untersuchten Gewebes möglichst präzise sicherstellen zu können. Optional kann das Raman-Spektrometer-System 1 auch mit einer Markierungseinheit bestückt werden, um das zu entfernende Gewebe, zum Beispiel auf der Haut oder dem jeweiligen Organ, zu markieren.

Das mobile Raman-Spektrometer-System 1 ist zudem mit einer Messeinrichtung 2 und einer damit verbundenen Auswerteeinheit 3 mit Auswerteprogramm 4 dargestellt. In der Figur 1 sind die Auswerteeinheit 3 mit Auswerteprogramm 4 dabei integraler Bestandteil eines Hauptkörpers 5 des mobilen Raman-Spektrometer-Systems 1. Dabei können diese Komponenten ausgebildet sein, flexibel austauschbar in diesem Hauptkörper 5 angeordnet zu sein. Bedienmodule der Auswerteeinheit 3 mit Auswerteprogramm 4 können zudem in einer nicht näher dargestellten Ausführungsvariante in hierfür vorgesehenen Aussparungen des Hauptkörpers 5 bedienerfreundlich vorgesehen sein. Auch können in einer nicht näher dargestellten Ausführungsvariante solche Bedienmodule separat vorgesehen sein und im Wesentlichen an einer Außenseite des Hauptkörper 5 angeordnet sein.

Der Hauptkörper 5 kann, wie dargestellt, eine im Wesentlichen rechteckige äußere Form aufweisen. Jegliche andere Formen sind vorstellbar. Auch ist vorstellbar, dass der Hauptkörper 5 in einer nicht näher dargestellten Ausführungsvariante mehrteilig ausgebildet ist, sodass dieser Hauptkörper 5 etwa für Transportzwecke oder zur Reinigung leicht zerlegbar ist.

Der Hauptkörper 5 kann beispielsweise partiell oder vollständig aus Edelstahl vorgesehen sein. Andere Materialien sind vorstellbar, wobei es von Vorteil ist, wenn diese Materialien mittels eines gängigen Sterilisierverfahrens zu reinigen sind. Beispielsweise ist es von Vorteil, wenn die Materialien so beschaffen sind, sodass sie in einem Autoklaven oder dergleichen gereinigt werden können und dabei innenliegende Komponenten des Systems 1 vor externen Einflüssen schützen.

Die Messeinrichtung 2 ist mittels einer ersten Verbindungsleitung 6 mit der Auswerteeinheit 3 mit Auswerteprogramm 4 gekoppelt dargestellt. Alternativ ist vorstellbar, dass die Messeinrichtung 2 mittels einer beliebigen Funkverbindung mit der Auswerteeinheit 3 mit Auswerteprogramm 4 gekoppelt vorgesehen ist.

In einer nicht näher dargestellten Ausführungsvariante ist zudem vorstellbar, dass die Messeinrichtung 2 und der Hauptkörper 5 mit den im Inneren des Hauptkörpers 5 angeordneten Komponenten des Systems 1 eine bauliche Einheit bilden und dabei entweder partiell oder insgesamt im Wesentlichen stabförmig ausgebildet sind.

In diesem Zusammenhang ist zudem vorstellbar, dass die Ausmaße so vorgesehen sind, sodass eine anwendende Person dieses System 1 dann bequem mit einer Hand umgreifen kann und beispielsweise gleich einem Stift für eine anstehende Untersuchung halten kann. Auch können die Ausmaße so vorgesehen sein, sodass eine entsprechende Haltevorrichtung zur Haltung anstelle einer Hand verwendet werden kann.

Die in der Figur 1 dargestellte Messeinrichtung 2 ist stabförmig ausgebildet und kann so ausgebildet sein, sodass eine Bedienung mit einer Hand von einem Anwender bequem möglich ist. In diesem Zusammenhang kann die Messeinrichtung 2 im Wesentlichen stabförmig ausgebildet sein und einen Umfang aufweisen, welcher mit einer Hand umgreifbar ist, wobei ein erster Endbereich ausgelegt ist, mit der zu bemessenden Probe in Kontakt gebracht zu werden, und ein zweiter Endbereich ausgelegt ist, wenigstens eine nicht näher dargestellte Strahlungsquelle, beispielsweise eine Laservorrichtung oder dergleichen, austauschbar im Inneren der Messeinrichtung 2 zu halten, sodass emittierte Strahlen der wenigstens eine Strahlungsquelle im Wesentlichen in Richtung erster Endbereich auslenkbar sind.

In einer nicht näher dargestellten Ausführungsvariante ist vorstellbar, dass die Messeinrichtung 2 weitere Verbindungsmittel aufweist, welche ausgelegt sind, die Messeinrichtung 2 mit der Auswerteeinheit 3 mit Auswerteprogramm 4 so zu koppeln, sodass die Messeinrichtung 2 separat von den restlichen Komponenten des mobilen Raman-Spektrometer-Systems 1 im Umkreis des mobilen Raman-Spektrometer-Systems 1 in einem Abstandsintervall von 0,1 bis 10 m, vorzugsweise von 0,5 bis 5 m, vorzugsweise von 1 bis 3 m, eingesetzt werden kann, sodass die mittels der Messeinrichtung 2 gemessenen Raman-Spektrometer-Parameter einer menschlichen, tierischen oder pflanzlichen Probe entsprechend in Bezug auf die restlichen Komponenten des mobilen Raman-Spektrometer-Systems 1 örtlich flexibel erhebbar sind.

Bezogen auf die Bildebene ist oberhalb des Hauptkörpers 5 und mit einer zweiten Verbindungsleitung 7 mit der Auswerteeinheit 4 mit Auswerteprogramm 5 gekoppelt eine Ausgabeeinheit 8 des mobilen Raman-Spektrometer-Systems 1 dargestellt. Diese Ausgabeeinheit 8 des mobilen Raman-Spektrometer-Systems 1 kann beispielsweise in Form eines handelsüblichen Monitorsystems oder dergleichen vorgesehen sein. Auch kann ein Monitorsystem eingesetzt werden, wie es üblicherweise in einem Operationssaal verwendet wird. Ebenso kann optional auch eine Übertragungseinheit vorgesehen sein, welche dann die aktuellen Bilder, beispielsweise in Echtzeit, an eine weitere medizinische Fachkraft, beispielsweise einem Pathologen, weiterleitet, wobei diese Person dann nicht direkt vor Ort sein muss, sondern sich beispielsweise sogar in einem anderen Land aufhält. Globale Kompetenzen von medizinischen Fachkräften lassen sich so mit Vorteil zusammenbringen, um bestmögliche Ergebnisse zu erzielen. Auch können somit Reisetätigkeiten dieser medizinischen Fachkräften weiter reduziert werden.

Auf einer Bildfläche 9 der Ausgabeeinheit 8 des mobilen Raman-Spektrometer-Systems 1 sind beispielhaft zwei Geometrien dargestellt, wobei vorstellbar ist, dass die rechteckige Geometrie beispielsweise krankes Gewebe einer zu bemessenden nicht näher dargestellten Probe ausweist und die kreisförmige Geometrie entsprechend gesundes Gewebe einer zu bemessenden nicht näher dargestellten Probe ausweist. Die Ausgabeeinheit 8 kann optional auch als kleinere Monitoreinheit vorgesehen sein, welche entsprechend an der Messeinrichtung 2 angeordnet vorgesehen ist. So kann ein Anwender alle wichtigen Aktionen in einem Blickfeld überschauen, ohne dass insbesondere bei kritischen Aktionen ein Blickfeld, etwa durch Kopfbewegungen oder Körperdrehungen, verändert werden müsste.

Die dargestellte Auswerteeinheit 3 mit Auswerteprogramm 4 ist insofern ausgelegt ist, die gemessenen Raman-Spektrometer-Parameter einer nicht näher dargestellten menschlichen, tierischen oder pflanzlichen Probe hinsichtlich wenigstens einer medizinischen Diagnose zu klassifizieren, sodass einem Anwender des mobilen Raman-Spektrometer-Systems 1 eine differenzierte Ansicht der Probe mittels der Ausgabeeinheit 8 bereitstellbar ist. Die differenzierte Ansicht kann dabei auch mittels der Ausgabeeinheit 8 so vollzogen werden, dass auf der Ausgabeeinheit 8 Markierungen vorgesehen werden können, um einen diagnostizierten Bereich einzugrenzen und gegebenenfalls jeweilige diagnostizierte Bereiche weiter zu unterscheiden.

Optional kann das mobile Raman-Spektrometer-System 1 in einer nicht näher dargestellten Ausführungsvariante ausgelegt sein, eine Vielzahl simultan auszuwertender Proben zu behandeln beziehungsweise zu messen, um beispielsweise die statistische Aussagekraft der Untersuchung zu verbessern.

Für diese Zwecke können beispielsweise auch zwei Messeinrichtungen 2 vorgesehen sein oder es kann eine Messeinrichtung 2 des mobilen Raman-Spektrometer-Systems 1 vorgesehen sein, welche entsprechend ausgelegt ist, benachbarte Bereiche am Gewebe zu bemessen oder zwei separate Proben von verschiedenen Organismen gleichzeitig zu bemessen, wobei diese in einem gewissen Mindestabstand zueinander zu platzieren wären.

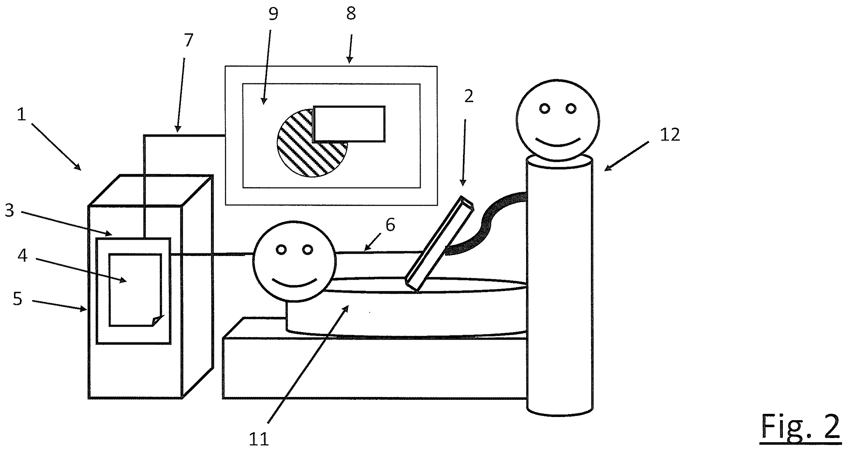

Fig. 2 zeigt eine schematische Darstellung eines Anwenderszenarios eines mobilen Raman-Spektrometer-Systems 1 nach einem Ausführungsbeispiel der vorliegenden Erfindung. Es kann sich beispielsweise um ein mobiles Raman-Spektrometer-System 1 gemäß dem in Figur 1 dargestellten handeln. Auch können die oben erwähnten nicht näher dargestellten Ausführungsvarianten des mobilen Raman-Spektrometer-Systems 1 entsprechend in Figur 2 vorgesehen sein.

Ein auf einem Behandlungstisch 10 liegender Patient 11 wird von einer danebenstehenden medizinischen Fachkraft 12 untersucht. Dabei hält die medizinische Fachkraft 12 eine stabförmige Messeinrichtung 2 des mobilen Raman-Spektrometer-Systems 1 direkt auf ein Gewebebereich des Patienten 11.

In diesem Fall kann es sich beispielsweise um einen Gewebebereich einer Hautpartie in Bauchnähe des Patienten 11 handeln. Vorstellbar sind jedoch auch Untersuchungen von anderen Gewebebereichen des Patienten 11. So ist beispielsweise auch eine partiell innerliche Untersuchung von Gewebebereichen, etwa der Mundschleimhaut oder dergleichen, vorstellbar.

Über eine erste Verbindungsleitung 6 werden die gemessenen Raman-Spektrometer-Parameter an eine Auswerteeinheit 4 mit Auswerteprogramm 5 des mobilen Raman-Spektrometer-Systems 1 übermittelt. Die Auswerteeinheit 3 mit Auswerteprogramm 4 ist dabei ausgelegt, die gemessenen Raman-Spektrometer-Parameter der hier menschlichen Probe hinsichtlich wenigstens einer medizinischen Diagnose zu klassifizieren, sodass der medizinischen Fachkraft 12, sprich dem Anwender des mobilen Raman-Spektrometer-Systems 1, eine differenzierte Ansicht der Probe mittels einer Ausgabeeinheit 8 bereitstellbar ist.

Während der Messung kann somit die medizinische Fachkraft 12 auf einer Bildfläche 9 der Ausgabeeinheit 8 die untersuchte Probe in einer klassifizierten Ansicht begutachten und anschließend entscheiden, ob und wo etwa Gewebe operativ oder per Schnitttechnik entfernt werden muss.

In diesem Zusammenhang ist vorstellbar, dass das dargestellte mobile Raman-Spektrometer-System 1 ausgelegt ist, sodass die Auswerteeinheit 3 mit Auswerteprogramm 4 für die Zwecke der Klassifizierung mit wenigstens einer nicht näher dargestellten Datenbank für die medizinische Diagnostik von menschlichen, tierischen oder pflanzlichen Proben basierend auf den Raman-Spektrometer-Parametern verbindbar ist.

Auch ist vorstellbar, dass diese Datenbank ein integraler Bestandteil des mobilen Raman-Spektrometer-Systems 1 ist. Auch ist vorstellbar, dass mehrere Datenbank gleichzeitig oder je nach Bedarf gekoppelt werden können. Auch ist vorstellbar, dass mehrere Datenbanken von verschiedenen mobilen Raman-Spektrometer-System 1 für jeweilige Klassifizierungszwecke bei einem jeweiligen mobilen Raman-Spektrometer-System 1 koppelbar sind, sodass über diese vernetzten Datenbanken eine noch zielsichere und effiziente Klassifizierung bewirkbar ist.

Auch ist vorstellbar, dass das Auswerteprogramm 4 mittels wenigstens einer benutzerdefinierten Eingabe anpassbar ist, sodass eine individualisierte Nutzung der jeweiligen gekoppelten Datenbank möglich ist. Solch eine wenigstens eine benutzerdefinierte Eingabe kann dabei ausgewählt sein aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden.

Solch eine Eingabe kann auch als Aktualisierungsvorgang der jeweils gekoppelten Datenbank aufgefasst werden. Auch ist vorstellbar, dass solch eine Vorgang zumindest teilweise durchführbar ist, sodass solch eine Aktualisierung beispielsweise im Hintergrund durchführbar ist, wobei eine benutzerdefinierte und somit eine Autorisierung von solch Eingaben vorstellbar ist.

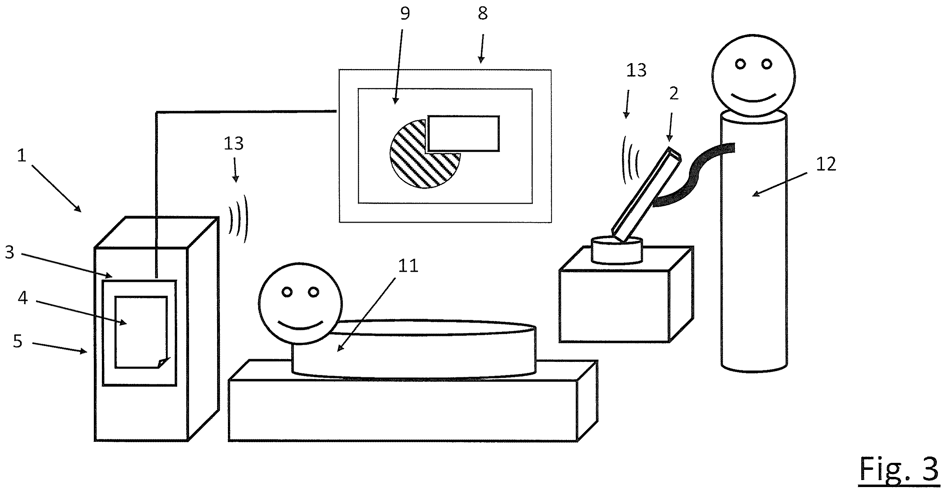

Fig. 3 zeigt schematische Darstellung eines weiteren Anwenderszenarios eines mobilen Raman-Spektrometer-Systems 1 nach einem Ausführungsbeispiel der vorliegenden Erfindung. Es kann sich beispielsweise um ein mobiles Raman-Spektrometer-System 1 gemäß dem in Figur 1 dargestellten handeln. Auch können die oben erwähnten nicht näher dargestellten Ausführungsvarianten des mobilen Raman-Spektrometer-Systems 1 entsprechend in Figur 3 vorgesehen sein.

In diesem Anwenderszenario ist eine medizinische Fachkraft 12 beabstandet von einem liegenden und zu untersuchenden Patienten 11 dargestellt, wobei diese medizinische Fachkraft 12 eine Messeinrichtung 2 eines mobilen Raman-Spektrometer-Systems 1 in der Hand hält, um eine zuvor von dem Patienten 11 entnommene Probe näher zu bemessen beziehungsweise zu untersuchen.

Die Messeinrichtung 2 eines mobilen Raman-Spektrometer-Systems 1 weist dabei nicht näher dargestellte weitere Verbindungsmittel zur Kopplung der Messeinrichtung 2 mit einer Auswerteeinheit 3 mit Auswerteprogramm 4 des mobilen Raman-Spektrometer-Systems 1 auf, welche ausgelegt sind, die Messeinrichtung 2 separat von den restlichen Komponenten des mobilen Raman-Spektrometer-Systems 1 im Umkreis des mobilen Raman-Spektrometer-Systems 1 in einem Abstandsintervall von 0,1 bis 10 m, vorzugsweise von 0,5 bis 5 m, vorzugsweise von 1 bis 3 m, einzusetzen, sodass die mittels der Messeinrichtung 2 gemessenen Raman-Spektrometer-Parameter der hier menschlichen Probe entsprechend in Bezug auf die restlichen Komponenten des mobilen Raman-Spektrometer-Systems 1 örtlich flexibel erhebbar sind.

Jeweilige Funkwellensymbole 13 stellen in der Figur 3 diese Kopplung zwischen Messeinrichtung 2 und der Auswerteeinheit 3 mit Auswerteprogramm 4 dar.

Insofern ist vorstellbar, dass die nicht näher dargestellten weiteren Verbindungsmittel derart vorgesehen sind, sodass jeweilige Sende/ Empfängermodule auf beiden Seiten so angeordnet werden, sodass diese Funkverbindung in einem Abstandsintervall von 0,1 bis 10 m, vorzugsweise von 0,5 bis 5 m, vorzugsweise von 1 bis 3 m einwandfrei bereitstellbar ist. Beispielsweise kann es sich bei den weiteren Verbindungsmitteln um jegliche gängige Funkverbindungstechniken für einen Transfer von erhobenen Messdaten handeln.

Insofern ist die dargestellte Messeinrichtung 2 ausgelegt, Raman-Spektrometer-Parameter der hier menschlichen Probe separat oder am Organismus zu messen, wobei hier eine separate Bemessung in räumlicher Nähe vollzogen wird.

In einer nicht näher dargestellten Ausführungsvariante ist vorstellbar, dass das Raman-Spektrometer-System 1 ausgelegt ist, eine Vielzahl von Proben simultan zu messen und zu klassifizieren.

Die dargestellte Messeinrichtung 2 ist im Wesentlichen stabförmig ausgebildet und weist einem Umfang auf, welcher mit einer Hand umgreifbar ist, wobei ein erster Endbereich ausgelegt ist, mit der zu bemessenden Probe in Kontakt gebracht zu werden, und ein zweiter Endbereich ausgelegt ist, wenigstens eine Strahlungsquelle austauschbar im Inneren der Messeinrichtung 2 zu halten, sodass emittierte Strahlen der wenigstens eine Strahlungsquelle im Wesentlichen in Richtung erster Endbereich auslenkbar sind.

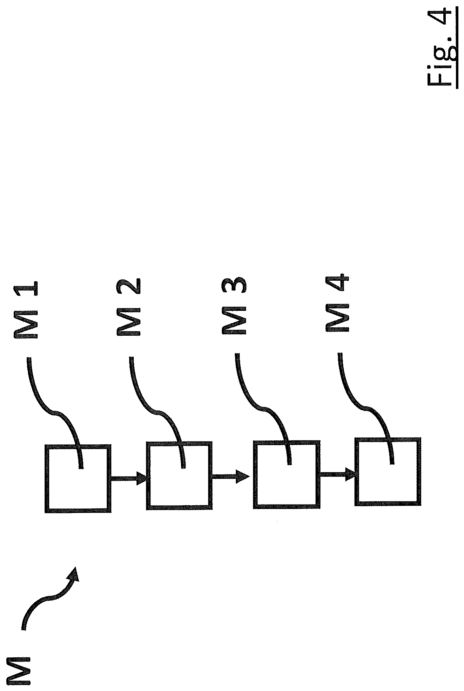

Fig. 4 zeigt ein schematisches Flussdiagramm für ein Verfahren für die medizinische Diagnostik von menschlichen, tierischen oder pflanzlichen Proben nach einem Ausführungsbeispiel der vorliegenden Erfindung. In einem ersten Verfahrensschritt M1 wird ein erfindungsgemäßes mobiles Raman-Spektrometer-System 1 bereitgestellt und aktiviert. In einem zweiten Verfahrensschritt M2 wird eine menschliche, tierische oder pflanzliche Probe mittels des mobilen Raman-Spektrometer-Systems 1 gemessen. In einem dritten Verfahrensschritt M3 werden Ergebnisse der Raman-Spektrometer-Messungen hinsichtlich wenigstens einer medizinischen Diagnose mittels des mobilen Raman-Spektrometer-Systems 1 klassifiziert. In einem vierten Verfahrensschritt M4 werden die klassifizierten Ergebnisse mittels des mobilen Raman-Spektrometer-Systems 1 ausgegeben, sodass einem Anwender des mobilen Raman-Spektrometer-Systems 1 eine differenzierte Ansicht der Probe mittels des mobilen Raman-Spektrometer-Systems 1 bereitstellbar ist.

### BEZUGSZEICHENLISTE

- 1: Raman-Spektrometer-System
- 2: Messeinrichtung
- 3: Auswerteeinheit
- 4: Auswerteprogramm
- 5: Hauptkörper
- 6: erste Verbindungsleitung
- 7: zweite Verbindungsleitung
- 8: Ausgabeeinheit
- 9: Bildfläche
- 10: Behandlungstisch
- 11: Patient
- 12: medizinische Fachkraft
- 13: Funkwellensymbol
- M: Verfahren
- M1: erster Verfahrensschritt
- M2: zweiter Verfahrensschritt
- M3: dritter Verfahrensschritt
- M4: dritter Verfahrensschritt

## Patentansprüche

1. Mobiles Raman-Spektrometer-System (1) für die medizinische Diagnostik von menschlichen, tierischen oder pflanzlichen Proben umfassend eine Messeinrichtung (2), eine damit verbundene Auswerteeinheit (3) mit Auswerteprogramm (4) und eine damit verbundene Ausgabeeinheit (8) **dadurch gekennzeichnet, dass** die Auswerteeinheit (3) mit Auswerteprogramm (4) ausgelegt ist, die gemessenen Raman-Spektrometer-Parameter einer menschlichen, tierischen oder pflanzlichen Probe hinsichtlich wenigstens einer medizinischen Diagnose zu klassifizieren, sodass einem Anwender des mobilen Raman-Spektrometer-Systems (1) eine differenzierte Ansicht der Probe mittels der Ausgabeeinheit (8) bereitstellbar ist.

2. Mobiles Raman-Spektrometer-System (1) nach Anspruch 1, wobei die Auswerteeinheit (3) mit Auswerteprogramm (4) für die Zwecke der Klassifizierung mit wenigstens einer Datenbank für die medizinische Diagnostik von menschlichen, tierischen oder pflanzlichen Proben basierend auf den Raman-Spektrometer-Parametern verbindbar ist oder wenigstens eine solche Datenbank umfasst.

3. Mobiles Raman-Spektrometer-System (1) nach Anspruch 2, wobei das Auswerteprogramm (4) mittels wenigstens einer benutzerdefinierten Eingabe anpassbar ist, sodass eine individualisierte Nutzung der jeweiligen Datenbank möglich ist.

4. Mobiles Raman-Spektrometer-System (1) nach Anspruch 3, wobei die wenigstens eine benutzerdefinierte Eingabe ausgewählt ist aus:
gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden, oder dergleichen, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.

5. Mobiles Raman-Spektrometer-System (1) nach einem der vorhergehenden Ansprüche, wobei die Messeinrichtung (2) ausgelegt ist, Raman-Spektrometer-Parameter der menschlichen, tierischen oder pflanzlichen Proben separat oder am Organismus zu messen.

6. Mobiles Raman-Spektrometer-System (1) nach einem der vorhergehenden Ansprüche, wobei das mobile Raman-Spektrometer-System (1) ein Energiesystem umfasst, welches ausgelegt ist, das Raman-Spektrometer-System (1) autark oder mittels eines externen Energieversorgungsnetzes mit elektrischer Energie zu versorgen.

7. Mobiles Raman-Spektrometer-System (1) nach einem der vorhergehenden Ansprüche, wobei das Raman-Spektrometer-System (1) im Wesentlichen Außenmaterialien, insbesondere Edelstahl, umfasst, welche für ein Sterilisationsverfahren geeignet sind, sodass das mobile Raman-Spektrometer-System (1) nach Durchlauf eines Sterilisierverfahrens in einem Operationssaal einsetzbar ist.

8. Mobiles Raman-Spektrometer-System (1) nach einem der vorhergehenden Ansprüche, wobei das Raman-Spektrometer-System (1) ausgelegt ist, eine Vielzahl von Proben simultan zu messen und zu klassifizieren.

9. Mobiles Raman-Spektrometer-System (1) nach einem der vorhergehenden Ansprüche, wobei das Raman-Spektrometer-System (1) ausgelegt ist, mittels wenigstens einem mit dem Raman-Spektrometer-System (1) koppelbaren externen Gerät gesteuert zu werden.

10. Mobiles Raman-Spektrometer-System (1) nach Anspruch 9, wobei das externe Gerät auswählbar ist aus: Smartphone, Tablet, Smartwatch, Laptop, Cloud-Anwendung.

11. Mobiles Raman-Spektrometer-System (1) nach einem der vorhergehenden Ansprüche, wobei die Messeinrichtung (2) weitere Verbindungsmittel zur Kopplung der Messeinrichtung (2) mit der Auswerteeinheit (3) mit Auswerteprogramm (4) umfasst, welche ausgelegt sind, die Messeinrichtung (2) separat von den restlichen Komponenten des mobilen Raman-Spektrometer-Systems (1) im Umkreis des mobilen Raman-Spektrometer-Systems (1) in einem Abstandsintervall von 0,1 bis 10 m, vorzugsweise von 0,5 bis 5 m, vorzugsweise von 1 bis 3 m, einzusetzen, sodass die mittels der Messeinrichtung (2) gemessenen Raman-Spektrometer-Parameter einer menschlichen, tierischen oder pflanzlichen Probe entsprechend in Bezug auf die restlichen Komponenten des mobilen Raman-Spektrometer-Systems (1) örtlich flexibel erhebbar sind.

12. Mobiles Raman-Spektrometer-System (1) nach einem der vorhergehenden Ansprüche, wobei die Messeinrichtung (2) im Wesentlichen stabförmig ausgebildet ist und einem Umfang aufweist, welcher mit einer Hand umgreifbar ist, wobei ein erster Endbereich ausgelegt ist, mit der zu bemessenden Probe in Kontakt gebracht zu werden, und ein zweiter Endbereich ausgelegt ist, wenigstens eine Strahlungsquelle austauschbar im Inneren der Messeinrichtung (2) zu halten, sodass emittierte Strahlen der wenigstens eine Strahlungsquelle im Wesentlichen in Richtung erster Endbereich auslenkbar sind.

13. Verfahren für die medizinische Diagnostik von menschlichen, tierischen oder pflanzlichen Proben umfassend die folgenden Schritte:
• Bereitstellen und Aktivieren (M1) eines mobilen Raman-Spektrometer-Systems (1) nach einem der Ansprüche 1 bis 10;
• Messen (M2) einer menschlichen, tierischen oder pflanzlichen Probe mittels des mobilen Raman-Spektrometer-Systems (1);
• Klassifizieren (M3) von Ergebnissen der Raman-Spektrometer-Messungen hinsichtlich wenigstens einer medizinischen Diagnose mittels des mobilen Raman-Spektrometer-Systems (1);
• Ausgeben (M4) der klassifizierten Ergebnisse mittels des mobilen Raman-Spektrometer-Systems (1), sodass einem Anwender des mobilen Raman-Spektrometer-Systems (1) eine differenzierte Ansicht der Probe mittels des mobilen Raman-Spektrometer-Systems (1) bereitstellbar ist.

14. Verfahren nach Anspruch 10, umfassend die folgenden weiteren Schritte:
• Verbinden der Auswerteeinheit (3) mit Auswerteprogramm (4) von dem mobilen Raman-Spektrometer-System (1) mit wenigstens einer Datenbank für die medizinische Diagnostik von menschlichen, tierischen oder pflanzlichen Proben basierend auf den Raman-Spektrometer-Parametern;
• Anpassen des Auswerteprogramms (4) mittels wenigstens einer benutzerdefinierten Eingabe, sodass eine individualisierte Nutzung der verbundenen externen Datenbank möglich ist.

15. Verfahren nach Anspruch 12, wobei die wenigstens eine benutzerdefinierte Eingabe ausgewählt ist aus: gendersensible Eingabe, Ernährungsangabe über menschlichen oder tierischen Probanden, Medikamenteneinnahme eines menschlichen oder tierischen Probanden, Düngemittelabgabe an pflanzlichen Probanden, Pflanzenschutzabgabe an pflanzlichen Probanden, oder dergleichen, wenigstens eine Information über korrelierende Einflüsse von Medikamenten und pathologischen Veränderungen bei wenigstens einem Organismus, Verdachtsdiagnosen, wenigstens eine Information aus einem Patientenregister aus wenigstens einem Land, wenigstens eine Information über eine Vorerkrankung des zu untersuchenden Organismus.
